# EUROPEAN PATENT SPECIFICATION

(11) **EP 1 481 056 B1**
(45) Date of publication and mention of the grant of the patent: **02.09.2009**
(21) Application number: 03706586.9
(22) Date of filing: 27.02.2003
(51) Int. Cl.: C12N 9/02, A61K 38/44, A61P 35/00

(54) **MODIFIED HUMAN MANGANESE SUPEROXIDE DISMUTASE AND USES THEROF**
MODIFIZIERTE HUMANE MANGAN-SUPEROXIDDISMUTASE UND DEREN VERWENDUNGEN
MANGANESE SUPEROXIDE DISMUTASE HUMAINE ET SES UTILISATIONS

(30) Priority: 28.02.2002 IT MI20020404
(43) Date of publication of application: 01.12.2004
(73) Proprietor: Mancini, Aldo, 80128 Napoli (IT)
(72) Inventor: MANCINI, Aldo, I-80128 Napoli (IT)
(74) Representative: Appoloni, Romano
(86) International application number: PCT/EP2003/002017
(87) International publication number: WO 2003/072768

(56) References cited:
- WO-A-93/18147
- WISPÉ, J.R. ET AL.: "Synthesis and processing of the precursor for human mangano-superoxide dismutase" BIOCHIMICA ET BIOPHYSICA ACTA, vol. 994, no. 1, 19 January 1989 (1989-01-19), pages 30-36, XP008020780 cited in the application -& DATABASE EMBL, HEIDELBERG, FRG [Online] 20 March 1987 (1987-03-20) WISPÉ, J.R. ET AL.: "Superoxide dismutase [Mn], mitochondrial precursor (EC 1.15.1.1)" Database accession no. P04179 XP002253346
- ZHONG, W. ET AL.: "Suppression of the malignant phenotype of human glioma cells by overexpression of manganese superoxide dismutase" ONCOGENE, vol. 14, no. 4, 30 January 1997 (1997-01-30), pages 481-490, XP002253344
- MONDOLA, P. ET AL.: "Evidence for Secretion of Cytosolic CuZnSuperoxide Dismutase by Hep G2 Cells and Human Fibroblasts" INTERNATIONAL JOURNAL OF BIOCHEMISTRY AND CELL BIOLOGY, vol. 28, no. 6, June 1996 (1996-06), pages 677-681, XP002253345
- MANCINI A. ET AL: 'Tumor suppressive activity of a variant isoform of manganese superoxide dismutase released by a human liposarcoma cell line' INT. J. CANCER vol. 119, 20 March 2006, pages 932 - 943

## Description

### State of the art

Superoxide radicals, highly reactive oxygen species, are generated in aerobic cells as refuse products of oxidative metabolism and, if accumulated in excessive amounts, can provoke irreversible damages to structures essential for cell function. In physiological conditions, cells are protected from these compounds by a group of metal proteins, the superoxide dismutase, that catalyze the dismutation of superoxide radicals to hydrogen peroxide that is converted to molecular oxygen by the action of Catalase. Various types of superoxide dismutase differ from each other in the metallic and/or proteic portion. In eukaryotic cells, the Copper/Zinc superoxide dismutase (Cu/Zn SOD), localized mainly in the cytoplasm, and the superoxide extracellular dismutase (ECSOD) and the Manganese Superoxide dismutase (MnSOD), with mostly mitochondrial localization, have been identified. The superoxide dismutaseare enzymes of remarkable pharmacological interest for their potential role in prevention and repair in all pathologies involving an oxidative damage. As an example, it has been proposed that these enzymes can be useful in prevention of tumors, in protection from damages deriving from reperfusion of ischemic tissues, in treatment of inflammatory states, in prevention and treatment of damages deriving from UV radiations, in reduction of cytotoxic and cardiotoxic effects of anti-tumor therapies.

For example Zhong W. et al. in Oncogene, 1997, 14: 481-490 described that the over-expression of MnSOD reduces the malignant phenotype in a human glioma cell line (U118) and Wispe J.R. et al. in Biochimica et Biophysica Acta, 1989, 30-36 describes recombinant expression of MnSOD cDNa and purification of MnSOD from rabbit liver.

The Manganese Superoxide Dismutase is of particular therapeutic interest for its localization in the mitochondria, known to represent the main endogenous source of superoxide radicals. The complete sequence of human manganese superoxide Dismutase cDNA has been identified in the eighties and is filed in SwissProt with accession number P04179 (Beck et al, Nucleic Acid Res. 16(13) 6224, 1988) and in GenBank under the accession number NM_00636 The cDNA encoded protein has been identified among others by Wispè et al. Biochim. Biophyis. Acta , 1989, 994: 30-36.

Subsequently numerous allelic variants of this enzyme have been described and are reviewed under the accession number in GenBank.

After gene identification, because of its potential therapeutic interest, several procedures have been established in order to obtain the human manganese superoxide dismutase by means of recombinant techniques, as for instance those described in patent EP0284105 or patent US 5246847.

In patent application WO 93/18147, submitted by the Applicant, a new cell line derived from human liposarcoma, called LSA, has been described, that produces a protein capable of selectively inhibiting cell division of estrogen sensitive epithelial tumor cells. Moreover, a new protein has been described, called p1LSA, with homologous aminoacidic sequence to that of the Hsp27 (Heat shock) protein.

### Summary of the invention

The authors of the present invention surprisingly found that a protein with cytotoxic activity against tumor cells can be purified from medium conditioned by LSA cells. The purified protein is recognized by anti-human manganese superoxide dismutase (hMnSOD) antibodies but unlike the wild type hMnSOD, is secreted rather than being mainly localized in mitochondria. In addition said protein is characterized by a MW different from that of the wild type protein and ranging from 28-33 kDa on SDS PAGE.

Moreover, the author of the present invention found that the new hMnSOD obtained from the cell culture medium, is not only capable of a selective and specific cytotoxic activity against tumor cells but stimulates healing of cutaneous lesions caused by chemical, physical or biological insults.

The present invention relates to a simple and inexpensive procedure to purify the aforementioned hMnSOD from conditioned medium from LSA cells by sequential steps of column chromatography.

Moreover, the present invention relates to pharmaceutical compounds containing the modified hMnSOD of the invention for prevention and therapy of tumors, for healing of ulcers and cutaneous lesions or for prevention and treatment of damages by ionizing radiation.

### Description of the figures

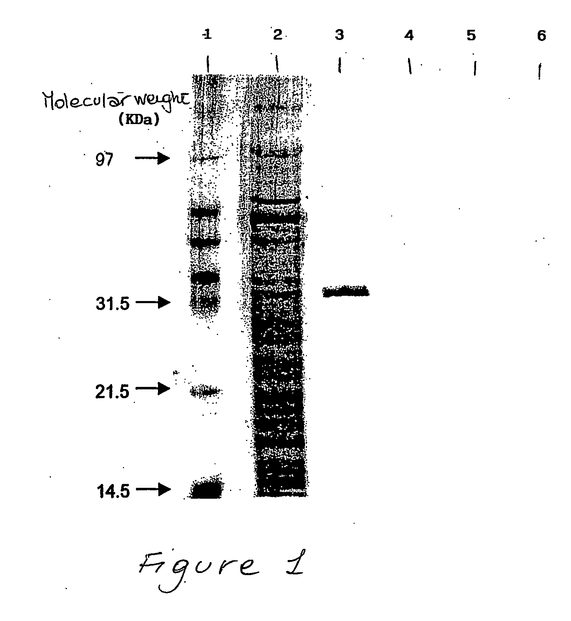

Figure 1 presents a SDS PAGE (lanes 1 and 2) and a Western Blotting (lanes 3 to 6) performed under reducing conditions and placed side by side. Gold-Comassie was used to stain proteins after SDS PAGE. Lane 1: molecular weight markers, lane 2: conditioned cell culture medium concentrated 100x in phosphate buffer pH 7,4 (Phosphate Saline Buffer). For Western Blotting, a sheep antibody anti-hMnSOD (Calbiochem) has been used for detection (lanes 3, 4, 5 and 6). Lanes 3 and 4: human modified MnSOD preparation purified according to the invention (lane 3) or after treatment with an antibody anti hMnSOD and protein-G-Sepharose (lane 4), lane 6: flow through from the second Q-Sepharose column, lane 7: flow through from Sephacryl-75 column.

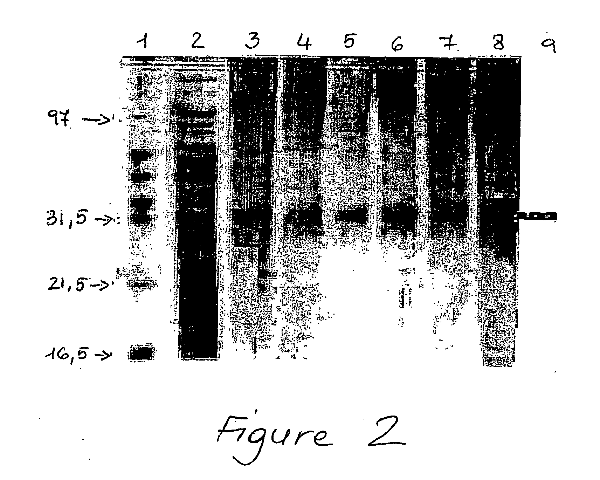

Figure 2: SDS-PAGE of FPLC chromatographically purified mhMnSOD. LSA conditioned supernatant was concentrated 10X and loaded on SDS-PAGE (lane 2); Lane 1: molecular weight markers MW corresponding, from top to bottom: 97, 31.5, 21.5,16.5. kDa. Lane 3. and 4: mhMnSOD as eluted after the 1^{st} chromatographic step (Q-Sepharose); lane 5 and 6 : mhMnSOD as eluted after the gel filtration (S-75) step; lane 7 and 8: mhMnSOD as eluted after the 2^{nd} chromatographic step (Q-Sepharose); lane 9: western blotting with a sheep anti-hMnSOD performed after chromatography.

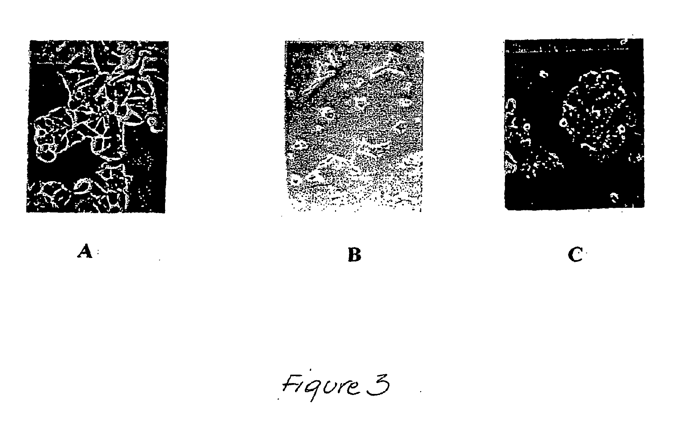

Figure 3. Biological characterization of the chromotographic fractions. MCF7 cells are shown after 24 hours incubation in presence of: (A) flow through from the second Q-Sepharose column (B) a sample from fractions containing the modified hMnSOD eluted from the second Q-Sepharose column (C) a sample from fractions eluted from the second Q-Sepharose column and not containing the hMnSOD.

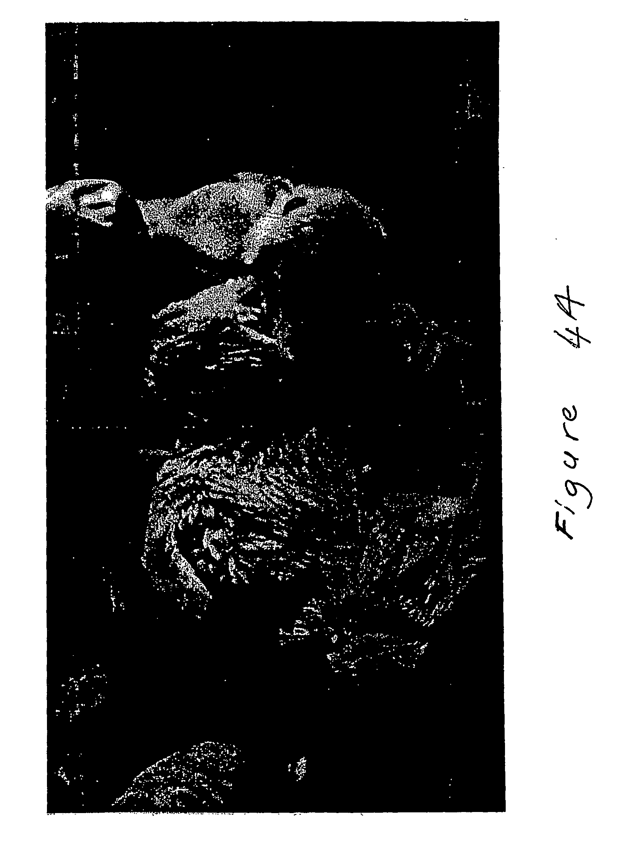

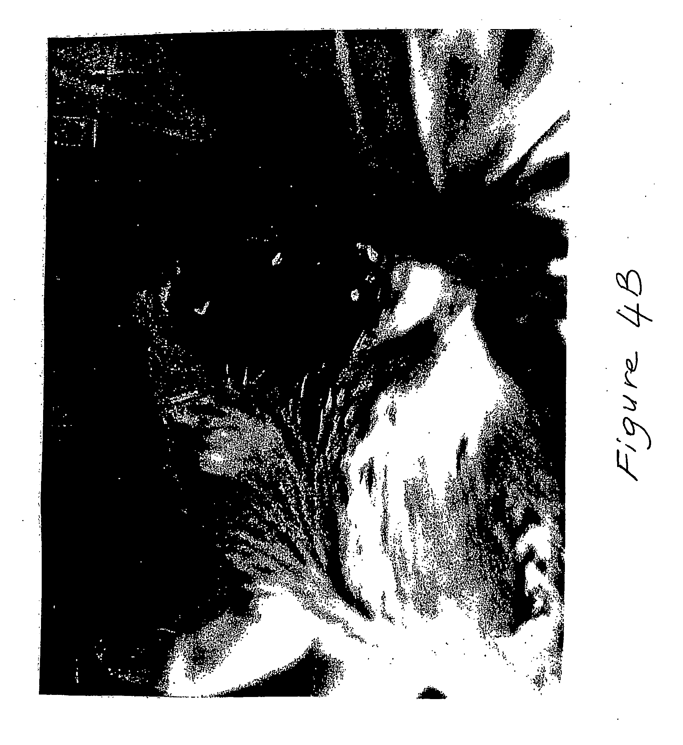

Figure 4. *In vivo* antitumor effect of the mhMnSOD. It is shown the difference in mammary tumor growth in an untreated BalbC-F-C3H mouse (upper panel A) or treated with the modified hMnSOD of the invention (lower panel B). In particular, an increase of the tumor mass from 1 to 4 cm diameter (panel A) did not occur when a tumor of the same initial diameter was treated for 15 days with the modified hMnSOD of the invention (panel B): tumor mass did not appear to increase and tumor tissue was completely necrotic.

### Detailed description of the invention

The present invention relates to the preparation of a modified human manganese superoxide dismutase that is secreted.

Herein "superoxide dismutase" designates an enzyme capable of catalyzing the dismutation reaction of superoxide radicals to hydrogen peroxide and molecular oxygen.

Herein "human manganese superoxide dismutase" designates a metalloprotein able to form complexes with manganese, composed of one or more polypeptide chains, wherein the monomer has the aminoacid sequence reported in the GenBank with the accession number P04179 (Beck et al, Nucleic Acid Res. 16(13) 6224, 1988) and in GenBank under the accession number NM_00636. The cDNA encoded protein has been identified among others by Wispè et al. Biochim. Biophyis. Acta, 1989, 994: 30-36. The mature form of this human MnSOD, hereinafter called hMnSOD or "wild type" hMnSOD has a Molecular weight of about 24 kDa.

"Modified human manganese superoxide dismutase" hereinafter called mhMnSOD, designates a human manganese superoxide dismutase containing structural modifications that allow it to be secreted, unlike the corresponding natural form and to display a wide range of activities described in details in the following application. The mhMnSOD is preferably oligomeric and the monomer is characterized by a molecular weight ranging from 28 and 33 kDa, preferably of about 30 kDa on a SDS PAGE.

Herein "natural form" or wild type hMnSOD designates a hMnSOD presenting the typical sub-cellular compartmentalization of the wild-type form, i.e. namely localized in mitochondria and herein after named hMnSOD.

In addition, as shown in Figure 2 and 3, the mhMnSOD of the invention is recognized by polyclonal antibodies against the human "wild-type" hMnSOD. The particular property of the mhMnSOD of the invention of being secreted in the culture medium makes possible to obtain large amounts by simple, fast and inexpensive purification procedures. In particular, the mhMnSOD of the invention is obtained by culturing LSA cells to subconfluency in serum-free medium lacking other proteins for at least 24 hours, preferably for at least one week, or even more preferably for at least 30 days, by collecting the conditioned medium, preferably on a daily basis and by replacing it with fresh culture medium followed by a step of purification wherein the mhMnSOD is separated from the conditioned medium by chromatographic techniques based on ion-exchange or immunoaffinity.

Preferably, the mhMnSOD purification comprises the following steps:
a) Concentration of cell culture medium in buffer having a pH ranging between 6 and 9 units;
b) application of the concentrated cell culture medium to an ionic exchange chromatography column having as stationary phase strong anionic exchanger group and elution with a linear salt gradient in buffer having pH from 6 to 9;
c) collection and identification of the fractions containing the modified human manganese superoxide dismutase;
d) pooling of the collected fractions and applying them to a gel filtration chromatographic column with stationary separating in the range of 20.000 to 40.000 Da, followed by elution with buffer having a pH range between 6 and 9;
e) collection and identification of the fractions containing the modified human manganese superoxide dismutase;
f) optionally further purifying the mhMnSOD by pooling of the collected fractions and applying them to an immunoaffinity chromatographic column with an anti-hMnSOD antibody bound to the stationary phase, followed by elution with a salt solution capable of disrupting the interaction between stationary phase with the antibody and the bound mhMnSOD in a buffer with pH from 6 to 9 and further identifying the MnSOD activity;
g) collection and identification of the fractions containing the modified human manganese superoxide dismutase.

According to an alternative purification procedure the step f) can be replaced by a step f') in which fractions containing the hMnSOD are applied to an ionic exchange chromatographic column witch strong anionic exchanger phase as stationary phase, and eluted with a discontinuous salt gradient in buffer having pH between 6 and 9.

In another- alternative procedure, the hMnSOD of the invention can be directly obtained from the LSA conditioned medium or from the active fractions eluted from the first chromatographic column by immunoaffinity chromatography with anti-human MnSOD antibodies. In the first case the conditioned medium can be directly applied to the chromatographic column without the concentration step. Moreover, the procedure of the invention can include, after purification, a step of de-salting and concentration by means of techniques known in the art, as for instance defiltration, dialysis and ultrafiltration. Preferably, dialysis is performed against an isotonic solution having pH 7.5, preferably PBS.

The LSA cell line has been deposited in the Deutsche Sammlung von Mikroorganismen und Zellkulturen GmbH (DSMZ) with accession number 2029. Preferably, in the step a) conditioned medium is concentrated between 10 and 100 times using techniques well known to one skilled in the art, for example ultrafiltration.

The stationary phase of the ionic exchange chromatographic columns used in the steps b) and f') is a matrix preferably having as a functional group a quaternary aminic group which is an anionic exchanger. Particularly preferred in said columns is the stationary phase represented by the commercial matrix denominated Q-Sepharose (Pharmacia). In the step d) the gel filtration chromatographic column contains a stationary phase preferably with a separation range between 3x10³ and 7x10⁴ Da, particularly the commercial matrix denominated Superdex 75 (Pharmacia).

In the step f) the immunoaffinity column contains as stationary phase a matrix capable of conjugating an antibody, preferably Protein-G-Sepharose or Sepharose preactivated with CnBr, for example CNBr-activated 4 Sepharose Fast Flow (Amersham Parmacia Biotech).

Preferably, a poyclonal antibody is bound to the stationary phase, for example the sheep anti-hMnSOD antibody produced from Calbiochem or other known anti-MnSOD antibodies.

Before applying the sample, all chromatographic columns used are pre-equilibrated with buffer having the same characteristics of the sample buffer and of the buffer that will be used for elution.

After sample application, columns are treated to eliminate non-specific interactions, for example by washing with several volumes of loading buffer, as known in the art.

Preferably in the steps a), b), d), f) and f) of the invention procedure, the buffers used have pH comprised between 7.4 and 8.

The elution from the first ionic exchange chromatographic column, in step b), is preferably performed using as eluent a linear gradient from 0.1 to 1 M of Sodium Chloride. Elution from the second ionic exchange chromatographic column in the step f) is preferably performed by a discontinuous gradient.

A particularly preferred discontinuous gradient is that obtained from the following elution buffers: A: TRIS 10 mM pH=8 + EDTA 1mM and B: Buffer A + NaCl 1M.

Preferably the elution is performed in the first 100 minutes with a gradient from 0 to 60% of buffer A and in the following 20 minutes with a gradient from 40 to 100% of buffer B.

Elution from the alternative immunoaffinity column is preferably performed by using a Sodium Chloride solution 0.5 M.

In the steps c) e) and g) the identification of fractions containing the hMnSOD can be performed by using immunological assays, known to skilled in the art, requiring an anti-hMnSOD antibody, as for example Western Blotting, while an example of enzymatic assay is the method of Winterbourn (Beyer W.F. et al. Phosphate inhibition of the Copper and Zinc containing Super-Oxide Dismutase: A re-examination Biochemistry, 1986, Vol. 25. pages. 6084-6088) based on the property of superoxide dismutase to inhibit reduction of "Nitro-blue of Tetrazolium" salt. Alternatively, the presence of mhMnSOD in the fractions eluted from chromatographic columns can be detected by biological assays, as those evaluating the cytotoxic activity of fractions on tumor cell lines like the human adenocarcinoma mammary MCF-7 cell line, primary tumor cells or human tumor cells from melanoma, gastric carcinoma, pancreatic carcinoma, ovarian carcinoma, pleuric mesothelioma, where activity indicates presence of hMnSOD.

As it will be described in more detail in examples that follow, the modified hMnSOD of the invention shows pharmacological activity of particular interest. Therefore, a further embodiment of the present invention relates to the pharmaceutical use of the modified hMnSOD comprising the preparation from LSA cell supernatant according to the procedure of the invention, for use as a medicament. Such mhMnSOD is recognized by antihMnSOD antibodies.

In particular, the mhMnSOD shows *in vitro* a selective cytotoxic activity against tumor cell lines and is capable of inducing necrosis of tumor masses *in vivo* very rapidly as demonstrated in a mouse model. Being its action specific only against tumor cells, one presumes that it completely lacks toxicity and that its potential adverse effects are lower than those induced by the known antitumor therapies.

The author has also found that the mhMnSOD is surprisingly capable, as a result of topical application, to induce healing of cutaneous lesions such as decubitus ulcers, torpid ulcers, cutaneous burns caused by chemical (acids or alkali) or physical (heat, ionizing radiation) agents. Therefore in a further embodiment the invention provides for the use of mhMnSOD prepared according to the invention to protect animal and humans from radiation, preferably from ionizing radiation.

In particular it has been found that the application of an ointment containing 10% p/p of hMnSOD of the invention for three times a day resulted in nearly complete healing of this type of lesions within less than thirty days. Moreover they said ointment proved to be particularly suitable for cosmetic use, being extremely effective in reducing cutaneous wrinkles, in reducing and neutralizing cutaneous inflammation, in reconstituting appropriate cutaneous and subcutaneous tonicity, in restoring skin firmness, in preventing possible cutaneous lesions, like for example chillblains.

The present invention refers therefore to the use of the modified hMnSOD of the invention for the preparation of a medicament for healing of cutaneous lesions such as decubitus ulcers, torpid ulcers and burns.

Moreover the hMnSOD of the invention has been proved to be effective in the treatment of individuals exposed to ionizing radiations and in preservation of organs for transplantation. Therefore the invention also refers to the use of said mhMnSOD to prepare a medicament for treatment of the aforesaid pathologies. The invention will be further explained by way of the following examples, to which, of course, is not limited.

### EXAMPLE1

### Isolation of modified human manganese superoxide dismutase from LSA cells conditioned culture medium.

1a) LSA cells were grown in Ham' s medium, containing F12, 5% of Fetal Calf Serum (FCS), until 80% confluent. After PBS washing, cells were incubated in F12 medium, without FCS or other mitogens, for approximately 30 days, during which cells continued to proliferate with a duplication time of 90 hours (the duplication time is of 48 hours in presence of serum)
   Starting from the fifth day of serum starvation, the conditioned medium, that contains proteins secreted from LSA cells, has been collected every day and replaced with fresh medium.
   Five liters of collected conditioned medium have been concentrated in PBS to pH 7,4 to a volume of 500 ml by ultrafiltration through Amicon filters with cut off of 3000 Da. The concentrated medium obtained by this procedure, has been analyzed by denaturing SDS PAGE. Numerous protein bands can be observed after gel staining with Gold-Comassie, as shown in Figure 1, lane 2.
   The concentrated medium has been applied to an ionic exchange column having Q-Sepharose as stationary phase and 1 cm length and 2 cm diameter, pre-equilibrated in TRIS 100mM pH 7.5.
   Elution has then been performed with 0.1-1 M Sodium Chloride gradient in 100mM TRIS buffer pH 7,5 at a flow rate of one ml per minute.
   1 ml fractions have been collected and assayed for cytotoxic activity on Mcf-7 cells from mammary adenocarcinoma as described in Example 2.
   Positive fractions, from number 31 to 35, have been pooled, applied to a gel filtration Superdex-75 (Pharmacia) chromatographic column of 2 cm diameter and 5 cm length and eluted with PBS buffer (phosphate buffer saline).
   1ml fractions have been collected and assayed for cytotoxic activity using the above assay. Positive fractions 11, 12, 13, have been pooled and a sample containing 30 µg of proteins was analyzed by SDS-PAGE under reducing conditions. By Gold-Comassie staining a heterogeneous band with sizes around 30 kDa was observed. A further anionic-exchange on Q sepharose was performed. The biologically active fractions were in addition blotted with an antihMnSOD antibody which reacted with the purified protein identifying the product as a modified hMnSOD with a MW higher than that of the known MnSOD (see fig 2 and fig 3).
   The C-terminal sequence of the protein cut out from the gel was analyzed by mass spectroscopy and by Edman degradation identifying the following sequence: AIWNVINWENVTER.
   Homology search in NCBI data bank has been performed using the software ProFound. The identified sequence corresponded to hMnSOD (accession number to the GenBank P04179) from amino acid 203 to amino acid 216.
   To separate the identified hMnSOD from other proteins, immunoaffinity chromatography has been performed using Protein G-Sepharose bound to a sheep anti-hMnSOD antibody (Calbiochem) as stationary phase, using a chromatographic column of 1 cm diameter and 2 cm length. After loading biologically active fractions, the column was washed with Tris buffer to eliminate non-specific interaction, and eluted with 0,5 M Sodium Chloride in Tris pH 7.5 bluffer. 1ml fractions have been collected and analyzed for cytotoxic activity. Active fractions were pooled and a sample containing approximately 30 µg of proteins has been analyzed by SDS PAGE as above. A single band of 30 kDa has been found.
1b) An alternative procedure to the one described in the Example 1a has been established wherein purification of mhMnSOD from the active gel filtration fractions was obtained by ionic exchange chromatography in discontinuous gradient (step gradient).

In detail, active fractions eluted from the gel filtration column have been applied to an ionic exchange chromatographic column having the same characteristics as the one pre-equlibrated in 10mM Tris pH 8 used in the Example 1 a.

The following buffer solutions were prepared:
A = 10 mM Tris pH 8 + 1 mM EDTA
B = Buffer A +1. M NaCl

Elution has been performed in the first 100 minutes with a linear gradient from 0% to 60% of A Buffer and of 100% to 40% of B Buffer. During the following 20 minutes elution has been instead performed with a linear gradient from 40% to 100% of B Buffer and from 60% to 0% of Buffer A.

1 ml fractions were collected and analyzed for cytotoxic activity.

Active fractions 13, 14 and 15, have been pooled.

In figure 2 and fig 3 is shown the SDS-PAGE and the western-blotting of purified mhMnSOD, of about 30 kDa.

### EXAMPLE 2

### Evaluation of the activity of the modified hMnSOD on tumor cell lines.

Fraction containing the mhMnSOD eluted from the ionic exchange column, as described in Example 1b, were pooled and analyzed for their cytotoxic activity on human mammary adenocarcinoma Mcf-7 cells, tumor cell lines derived from other organs such as lung, pancreas, stomach, pleuric mesothelium and melanoma, and on non tumor cell lines such as human epithelium cells Mcf 10, fibroblasts (POF) and keratinocytes (HaCat).

In each well, 50 µl of the pooled fractions containing hMnSOD were used in a volume of 250 µl, corresponding to a final concentration of hMnSOD between 10 and 50 µg/ml.

Flow-through from the second ionic exchange chromatographic column, and a sample of eluted fractions lacking manganese superoxide dismutase were used as control.

Figure 3 shows the results obtained in MCF7 cells, showing no effect in cells treated with flow-through from the second ionic exchange column (panel A), or with fractions not containing manganese superoxide dismutase eluted from the same column (panel C); white pooled fractions containing manganese superoxide dismutase exerted cytotoxicity on Mcf-7 cells (panel B). As further control that the cytotoxic effect was due to mhMnSOD, 100 µl of the superoxide dismutase solution have been mixed to 30 µl of a sheep anti-hMnSOD antibody (Calbiochem). 50 µl of Protein-G-Sepharose resin pre-equitibrated in PBS pH7.5 were then added to the mixture and incubated for three hours at room temperature under continuous rotatory shaking. Supernatant was then recovered by centrifugation. A sample of supernatant was then analyzed by Western blot, using for detection sheep anti-hMnSOD antibody (Calbiochem). As illustrated in Figure 1, lane 3, the supernatant was totally depleted of mhMnSOD. The supernatant was not toxic to Mcf-7 cells, indicating that the biologically active and toxic molecule was indeed the modified hMnSOD.

### EXAMPLE 3

### Evaluation of antitumor activity of modified hMnSOD in vivo.

The anti-tumor activity of the hMnSOD shown in Example 2 has been estimated on Balb-c-fC-3H mice bearing mammary carcinomas induced with Bittner virus In detail, mice were subcutaneously injected for 10 days in the peritumor region with 200 µl of either sterile solution containing 25 ng/ml of hMnSOD or, as control, with 200 µl of physiological solution. After 15 days all mice were sacrificed by cervical dislocation and examined by necroscopy. At the end of the experiment, mammary tumors appeared completely necrotic in all treated mice (see figure 4, panel B) and no metastasis was present in the lung. Instead in the untreated mice the tumor masses grew up to four fold the initial size (see figure 4, panel A) and large metastatic foci were found in the lungs.

Ultrasound was also performed on all animals in order to monitor density variations in tumor masses during treatment.

Results from this exam showed that tumor masses underwent liquefaction in treated animals unlike untreated animals where they remained solid and without necrosis.

### EXAMPLE 4

### in vitro identification of synergistic cytotoxic activity between modified hMnSOD and Cisplatin

Growth curves in Mcf-7 cells have been performed to compare the cytotoxic activity of hMnSO to that of Cisplatin. In detail, Mcf-7 cells have been treated either with Cisplatin (25 µgM) or hMnSOD (25 ng/ml), or with a mixture of Cisplatin (25µM) and hMnSOD (25ng/ml).

Cells treated with Cisplatin alone or with hMnSOD alone died within 5-6 days. Instead cells treated with both Cisplatin and hMnSOD died in just 24 hours. Noticeable, no resistant clone originated in the latter experiment or when the two molecules were individually used. Obtained results indicate the presence of a synergistic cytotoxic activity between the two molecules.

### EXAMPLE 5

### Composition for topical preparations

10 mg of hMnSOD purified from LSA cells conditioned culture medium are diluted and mixed, under shaking, with 1 Kg of hydrophilic vaseline. A cream prepared by this procedure is then divided in 100 g aliquots and stored in suitable containers. This cream is stable at room temperature and active for six months.

## Claims

1. A method for preparation of a secreted human manganese dismutase (hMnSOD), comprising the following steps:
I) growth of LSA cells (deposit number DSMZ 2029) in protein free medium for at least 24 hours and collection of the conditioned medium;
11) purification of the human manganese superoxide dismutase from the conditioned medium by immunoaffinity chromatography with an anti-hMnSOD antibody.

2. A method according to claim 1 wherein said step 11) comprises the following operations:
a) optional concentration of the LSA cells conditioned medium in a buffer with pH from 6 to 9;
b) application of the concentrated conditioned medium on an ionic exchange chromatographic column with a strong anionic exchanger as stationary phase and elution with linear salt gradient in buffer having pH between 6 and 9;
c) identification and collection of fractions containing the modified human manganese superoxide dismutase;
d) pooling of the collected fractions and loading on a gel filtration chromatographic column with stationary phase having a separation range of 20.000 to 40.000 Da, and elution with a buffer having pH between 6 and 9;
e) identification and collection of the fractions containing the human manganese superoxide dismutase;
f) loading of the fractions on an immunoaffinity chromatographic column with an antibody anti-hMnSOD bound to the stationary phase and elution of the secreted hMnSOD with a salt solution capable of disrupting the interaction between the antibody on the stationary phase and the bound hMnSOD in a buffer with pH from 6 to 9;

3. A method according to claim 2 further comprising de-salting and concentration.

4. A method according to claims 2 and 3, in which in step a) the medium is concentrated 10 to 100 times.

5. A method according to claims 2 - 4, in which in steps b) said anionic exchanger on the stationary phase has a quaternary amine group as functional group.

6. A method according to claim 5 wherein said stationary phase is Q-Sepharose.

7. A method according to claims from 2 to 6 wherein in step b) said linear salt gradient is a linear gradient ranging from 0.1 M to 1 M of Sodium Chloride.

8. A method according to claims 2-7, wherein in step d) the stationary phase has a separation range between 3 x10³ and 7 x10⁴Da.

9. A method according to claim 8, wherein said stationary phase is Superdex 75.

10. A method according to claims 2 -9, wherein in step f) the stationary phase is Protein-G-Sepharose or Sepharose preactivated with CnBr.

11. A method according to claims 2 to 14, wherein in step f) the antibody bound to the stationary phase is a polyclonal antibody.

12. A method according to claim 11, wherein in step g) the elution is performed with NaC10.5M.

13. A method according to claims 2 to 12, wherein in steps a), b), d) and g) said buffer has pH comprised from 7.4 to 8.

14. A method according to claims from 2 to 13, where in steps c), and e) the identification of fractions containing the human manganese superoxide dismutase is performed by immunological, biological or enzymatic assays.

15. A method according to claims 14, wherein said immunological assay is performed by Western blotting.

16. The method according to claim 15, wherein said biological test consists in testing the obtained fractions for cytotoxic activity on tumor cells, wherein said activity indicates the presence of a human superoxide dismutase.

17. The method according to claim 16, wherein said cell line is the adenocarcinoma Mcf-7 cell line.

18. A method according to claim 14, wherein said enzymatic assay consists in testing the obtained fractions for their ability to inhibit reduction of Nitro blue Tetrazolium salt.

19. A method for the preparation of a medicament for healing cutaneous lesions comprising the preparation of a secreted human manganese dismutase (hMnSOD) according to claims 1- 18.

20. A method according to claim 19 wherein said cutaneous lesions are decubitus ulcers, torpid ulcers and burns.

## Patentansprüche

1. Verfahren für die Herstellung einer sezernierten humanen Mangan-Dismutase (hMnSOD), umfassend die folgenden Schritte:
I) Wachstum von LSA-Zellen (Depotnummer DSMZ 2029) in proteinfreiem Medium für mindestens 24 Stunden und Abnahme des konditionierten Mediums;
II) Reinigung der humanen Mangan-Superoxiddismutase aus dem konditionierten Medium mithilfe von Immunaffinitätschromatographie mit einem anti-hMnSOD-Antikörper.

2. Verfahren gemäß Anspruch 1, wobei der genannte Schritt II) die folgenden Abläufe umfasst:
a.) optionales Aufkonzentrieren des konditionierten Mediums der LSA-Zellen in einem Puffer mit einem pH-Wert von 6 bis 9;
b.) Aufbringen des konzentrierten konditionierten Mediums auf eine Ionenaustausch-Chromatographiesäule mit einem starken Anionenaustauscher als stationärer Phase und Elution mit einem linearen Salzgradienten in einem Puffer mit einem pH-Wert zwischen 6 und 8;
c.) Identifizieren und Sammeln der Fraktionen, die die modifizierte humane Mangan-Superoxiddismutase enthalten;
d.) Poolen der gesammelten Fraktionen und Aufbringen auf eine Gelfiltrationschromatographiesäule mit einer stationären Phase mit einem Trennbereich von 20.000 bis 40.000kDa und Elution mit einem Puffer mit einem pH-Wert zwischen 6 und 8;
e.) Identifizieren und Sammeln der Fraktionen, die die humane Mangan-Superoxiddismutase enthalten;
f.) Aufbringen der Fraktionen auf eine Immunaffinitätschromatographiesäule mit einem anti-hMnSOD-Antikörper, der an die stationäre Phase gebunden ist, und Elution der sezernierten hMnSOD mit einer Salzlösung, mit welcher die Interaktion zwischen dem Antikörper auf der stationären Phase und der gebundenen hMnSOD in einem Puffer mit einem pH-Wert von 6 bis 8 gestört wird.

3. Verfahren gemäß Anspruch 2, welches ferner einen Entsalzungs- und Konzentrationsschritt umfasst.

4. Verfahren gemäß Anspruch 2 und Anspruch 3, wobei in Schritt a) das Medium 10-fach bis 100-fach aufkonzentriert wird.

5. Verfahren gemäß Ansprüchen 2-4, wobei in Schritt b) der genannte Anionenaustauscher auf der stationären Phase eine quaternäre Amingruppe als funktionelle Gruppe aufweist.

6. Verfahren gemäß Anspruch 5, wobei die genannte stationäre Phase Q-Sepharose ist.

7. Verfahren gemäß den Ansprüchen 2 bis 6, wobei in Schritt b) der lineare Salzgradient ein linearer Gradient im Bereich von 0,1 M bis 1M Natriumchlorid ist.

8. Verfahren gemäß den Ansprüchen 2-7, wobei in Schritt d) die stationäre Phase einen Trennbereich zwischen 3x10³ und 7 x 10⁴ Da aufweist.

9. Verfahren gemäß Anspruch 8, wobei die genannte stationäre Phase Superdex 75 ist.

10. Verfahren gemäß den Ansprüchen 2-9, wobei in Schritt f) die stationäre Phase Protein-G-Sepharose oder Sepharose, voraktiviert mit CnBr, ist.

11. Verfahren gemäß den Ansprüchen 2 bis 10, wobei in Schritt f) der an die stationäre Phase gebundene Antikörper ein polyklonaler Antikörper ist.

12. Verfahren gemäß Anspruch 11, wobei in Schritt g) die Elution mit NaC1 0,5 M durchgeführt wird.

13. Verfahren gemäß den Ansprüchen 2 bis 12, wobei in den Schritten a), b.), d) und g) der genannte Puffer einen pH-Wert im Bereich von 7,4 bis 8 umfasst.

14. Verfahren gemäß den Ansprüchen von 2 bis 13, wobei in den Schritten c) und e) die Identifikation der Fraktionen, welche die humane Mangan-Superoxiddismutase enthalten, mithilfe immunologischer, biologischer oder enzymatischer Assays durchgeführt wird.

15. Verfahren gemäß Anspruch 14, wobei der genannte immunologische Assay mit einem Western-Blot durchgeführt wird.

16. Verfahren gemäß Anspruch 15, wobei der genannte biologische Test im Testen der erhaltenen Fraktionen auf zytotoxische Aktiviät in Bezug auf Tumorzellen besteht, wobei die genannte Aktivität auf die Anwesenheit einer humanen Superoxiddismutase hindeutet.

17. Verfahren gemäß Anspruch 16, wobei die genannte Zelllinie die Adenokarzinom-Mcf-7-Zelllinie ist.

18. Verfahren gemäß Anspruch 14, wobei der genannte enzymatische Assay im Testen der erhaltenen Fraktionen auf ihre Fähigkeit, die Reduktion von Nitroblautetrazoliumsalz zu hemmen, besteht.

19. Verfahren für die Herstellung eines Arzneimittels zur Heilung kutaner Läsionen, umfassend die Herstellung einer sezernierten humanen Mangan-Dismutase (hMnSOD) gemäß den Ansprüchen 1-18.

20. Verfahren gemäß Anspruch 19, wobei diese kutanen Läsionen Dekubitus, Ulkus, torpiden Ulkus und Verbrennungen sind

## Revendications

1. Procédé pour la préparation d'une dismutase à manganèse humaine secrétée (hMnSOD), comportant les étapes suivantes :
I) croissance de cellules LSA (numéro de dépôt DSMZ 2029) dans un milieu exempt de protéines pendant au moins 24 heures et collecte du milieu conditionné,
II) purification de la superoxyde dismutase à manganèse humaine à partir du milieu conditionné par chromatographie d'immunoaffinité à l'aide d'un anticorps anti-hMnSOD.

2. Procédé selon la revendication 1, dans lequel ladite étape II) comporte les opérations suivantes :
a) concentration optionnelle du milieu conditionné par les cellules LSA dans un tampon ayant un pH de 6 à 9,
b) application du milieu conditionné concentré sur une colonne chromatographique échangeuse d'ions pourvue d'un puissant échangeur anionique en tant que phase stationnaire et élution avec un gradient linéaire de sel dans un tampon ayant un pH entre 6 et 9,
c) identification et collecte de fractions contenant la superoxyde dismutase à manganèse humaine modifiée,
d) regroupement des fractions collectées et chargement sur une colonne chromatographique de filtration sur gel dotée d'une phase stationnaire ayant une plage de séparation de 20 000 à 40 000 Da, et élution avec un tampon ayant un pH entre 6 et 9,
e) identification et collecte des fractions contenant la superoxyde dismutase à manganèse humaine,
f) chargement des fractions sur une colonne chromatographique d'immunoaffinité avec un anticorps anti-hMnSOD fixé sur la phase stationnaire et élution de la hMnSOD secrétée avec une solution saline capable de rompre l'interaction entre l'anticorps sur la phase stationnaire et la hMnSOD fixée dans un tampon ayant un pH de 6 à 9.

3. Procédé selon la revendication 2, comportant également une désalinisation et une concentration.

4. Procédé selon les revendications 2 et 3, dans lequel à l'étape a), le milieu est concentré 10 à 100 fois.

5. Procédé selon les revendications 2 à 4, dans lequel à l'étape b), ledit échangeur anionique sur la phase stationnaire possède un groupe amine quaternaire en tant que groupe fonctionnel.

6. Procédé selon la revendication 5, dans lequel ladite phase stationnaire est la Q-Sepharose.

7. Procédé selon les revendications 2 à 6, dans lequel à l'étape b), ledit gradient linéaire de sel est un gradient linéaire variant de 0,1 M à 1 M de chlorure de sodium.

8. Procédé selon les revendications 2 à 7, dans lequel à l'étape d), la phase stationnaire a une plage de séparation entre 3×10³ et 7×10⁴ Da.

9. Procédé selon la revendication 8, dans lequel ladite phase stationnaire est du Superdex 75.

10. Procédé selon les revendications 2 à 9, dans lequel à l'étape f), la phase stationnaire est de la protein-G-Sepharose ou de la Sepharose préactivée avec du CnBr.

11. Procédé selon les revendications 2 à 10, dans lequel à l'étape f), l'anticorps fixé sur la phase stationnaire est un anticorps polyclonal.

12. Procédé selon la revendication 11, dans lequel à l'étape g), l'élution est réalisée avec du NaCl 0,5 M.

13. Procédé selon les revendications 2 à 12, dans lequel aux étapes a), b), d) et g), ledit tampon a un pH compris entre 7,4 et 8.

14. Procédé selon les revendications 2 à 13, dans lequel aux étapes c) et e), l'identification de fractions contenant la superoxyde dismutase à manganèse humaine est réalisée par des analyses immunologiques, biologiques ou enzymatiques.

15. Procédé selon la revendication 14, dans lequel ladite analyse immunologique est réalisée par buvardage de western.

16. Procédé selon la revendication 15, dans lequel ledit test biologique consiste à tester les fractions obtenues pour leur activité cytotoxique sur des cellules tumorales, ladite activité indiquant la présence d'une superoxyde dismutase humaine.

17. Procédé selon la revendication 16, dans lequel ladite lignée cellulaire est la lignée cellulaire Mcf-7 d'adénocarcinome.

18. Procédé selon la revendication 14, dans lequel ladite analyse enzymatique consiste à tester les fractions obtenues pour déterminer leur capacité à inhiber une réduction du sel de nitro-bleu de tétrazolium.

19. Procédé pour la préparation d'un médicament destiné à guérir des lésions cutanées, comportant la préparation d'une dismutase à manganèse humaine secrétée (hMnSOD) selon les revendications 1 à 18.

20. Procédé selon la revendication 19, dans lequel lesdites lésions cutanées sont des escarres de décubitus, des ulcères torpides et des brûlures.
